# EUROPEAN PATENT SPECIFICATION

(11) **EP 4 067 501 B1**
(45) Date of publication and mention of the grant of the patent: **06.11.2024**
(21) Application number: 21870546.5
(22) Date of filing: 07.09.2021
(51) Int. Cl.: C12Q 1/6813

(54) **COMBINED PRODUCT FOR TESTING DNA**
KOMBINIERTES PRODUKT ZUR TESTUNG VON DNA
PRODUIT COMBINÉ POUR TESTER L'ADN

(30) Priority: 08.02.2021 CN 202110170603
(43) Date of publication of application: 05.10.2022
(73) Proprietor: Guangzhou Pluslife Technology Co., Ltd., Guangzhou, Guangdong 510663 (CN); Guangzhou Pluslife Biotech Co., Ltd., Guangzhou, Guangdong 510663 (CN)
(72) Inventor: XIAO, Zhuo, Guangzhou Guangdong 510663 (CN); FENG, Yaoheng, Guangzhou Guangdong 510663 (CN); RUAN, Mingxian, Guangzhou Guangdong 510663 (CN); CHEN, Chong, Guangzhou Guangdong 510663 (CN)
(74) Representative: J A Kemp LLP
(86) International application number: PCT/CN2021/116861
(87) International publication number: WO 2022/166196

(56) References cited:
- CN-A- 1 091 831
- CN-A- 1 865 934
- CN-A- 104 946 634
- CN-A- 109 750 091
- CN-A- 112 852 929
- CN-A- 112 961 943
- CN-A- 113 073 152
- US-A1- 2012 052 492
- US-A1- 2013 177 906
- US-A1- 2013 210 655
- US-B2- 9 587 280
- HARVEY, J.J. ET AL.: "SNP Analysis Using CataCleave Probes", JOURNAL OF CLINICAL LABORATORY ANALYSIS, vol. 22, 31 December 2008 (2008-12-31), pages 192 - 203, XP055098111, DOI: 10.1002/jcla.20240

## Description

### TECHNICAL FIELD

The present disclosure relates to the field of biotechnology, in particular, to a combination product for detecting DNA.

### BACKGROUND

After decades of development and rapid maturity in recent years, nucleic acid detection technology has been widely used in various aspects, such as infectious disease pathogen detection (including bacteria, mycoplasma, viruses, etc.), tumor mutation detection, genetic disease detection, and individual genotypes detection, etc. The nucleic acid detection technology has much better sensitivity and specificity in many application fields, such as infectious disease diagnosis, than colloidal gold/immunofluorescence method, and it is the gold standard for various diagnoses. Among the numerous nucleic acid detection techniques, the probe hybridization-based detection technology is well known and can be used in combination with a variety of techniques

US 2012/052492 discloses RNA/DNA hybrid probes used in combination with RNase H II in different assay formats.

The existing probes usually have no capability of signal amplification, but problems such as inconvenient detection, low sensitivity, and so on.

### SUMMARY

The present disclosure relates to a combination product for DNA detection, including a ribonuclease HII and a probe,
wherein the probe is a single-stranded probe, and has a sequence which can be partially or entirely complementary to a target DNA molecule to be detected, and one or more RNA bases are embedded in a complementary region of the probe and divide the complementary region of the probe into at least two segments, each of which independently has 0, 1, 2, 3, 4, 5, 6, 7, 8, 9, 10, 11, 12 or 13 DNA bases and base substitutions that do not interfere with a hybridization of a nucleic acid strand in total.

The present disclosure also relates to a kit including the combination product as described above.

The present disclosure also relates to a use of the combination product as described above in the detection of SNP, wherein the probe has one RNA base.

The present disclosure also relates to a use of the combination product as described above in the detection of a target DNA obtained from amplification with a LAMP, NEAR or RPA primer.

The beneficial effects of the present disclosure are as follows:
(1) the cleaved probes have an increased percentage in the complementary strand at the same concentration and thus an intensified signal;
(2) the product fragment is shorter after cleavage with the probe (≤13 bases), and easier to dissociate from the complementary strand, avoiding extension of the cleaved product as a primer and blockage of the complementary strand, so that the complementary strand binds to a new probe, thereby improving the sensitivity of the system;
(3) the system with good compatibility may be used in combination with various isothermal amplification methods such as LAMP, NEAR, RPA, etc., and can be incorporation with a reporting system before the reaction, so as to achieve result output without additional operations and reactions; and
(4) in contrast with fluorescent dyes and color indicators commonly used in other isothermal amplification, the probe can ensure the specificity for the detection result by identifying the amplification product when used in combination with isothermal amplification methods such as LAMP, NEAR, and RPA, etc.

### BRIEF DESCRIPTION OF THE DRAWINGS

To describe the technical solutions of the embodiments of the present disclosure or the prior art more clearly, the following briefly introduces the accompanying drawings required for describing the embodiments or the prior art. Apparently, the accompanying drawings in the following description show some embodiments of the present disclosure, and persons of ordinary skill in the art may also derive other drawings from these accompanying drawings without creative efforts.
Fig. 1 is a graph showing the results of the effect of the Tm value of a probe on detection in an embodiment of the present disclosure, wherein A represents the result of the reaction at 60°C, and B represents the result of the reaction at 65°C.
Fig. 2 is a graph showing the results of the effect of DNA base segment length on cleavage in an embodiment of the present disclosure, wherein panel A represents the result in a system without a polymerase, and panel B represents the result in a system with a polymerase.
Fig. 3 is a graph showing the results of the effect of DNA base segment length on the reportable temperature range in an embodiment of the present disclosure.
Fig. 4 is a graph showing the detection results of real-time fluorescence detection of SARS-CoV-2 in an embodiment of the present disclosure, wherein A represents the detection result in the RNase HII reporting system, and B represents the detection result by the dye method.
Fig. 5 is a graph showing the detection results of real-time fluorescence detection of SARS-CoV-2 in an embodiment of the present disclosure, wherein panel A represents the sensitivity detection result of a probe reporting system with a DNA maximum spacer segment of 12 bases, and panel B represents the sensitivity detection result in a probe reporting system with a maximum DNA spacer segment of 6 bases.
Fig. 6 is a graph showing the detection results of colloidal gold detection of SARS-CoV-2 (line display method) in an embodiment of the present disclosure, wherein A represents a negative control, and B represents a SARS-CoV-2 sample.
Fig. 7 is a graph showing the detection results of colloidal gold detection of SARS-CoV-2 (line elimination method) in an embodiment of the present disclosure, wherein A represents a negative control, and B represents a SARS-CoV-2 sample.
Fig. 8 shows the test result of the effect of the probe length on the identification of a single base in an embodiment of the present disclosure.
Fig. 9 is a graph showing the detection results of real-time fluorescence detection of SNP site at rs1815739 in an embodiment of the present disclosure, wherein A represents the detection result for a TT genotype sample, B represents the detection result for a CC genotype sample, and C represents the detection result for a CT genotype sample.
Fig. 10 is a graph showing the detection results of colloidal gold detection of SNP site at rs1815739 in an embodiment of the present disclosure, wherein F, G represent the detection results for TT genotype samples, C, D, and E represent the detection results for CC genotype samples, and A, B and H represent the detection result for CT genotype samples.

### DETAILED DESCRIPTION OF THE EMBODIMENTS

All technical and scientific terms used herein have the same meaning as commonly understood by skilled person in the art to which this disclosure belongs, unless otherwise defined. The terms used in the specification of the present disclosure herein are for the purpose of describing specific embodiments only and are not intended to limit the present disclosure. The term "and/or" used herein includes any and all combinations of one or more of the associated listed items.

The present disclosure relates to a combination product for DNA detection, including a ribonuclease HII and a probe, wherein, the probe is a single-stranded probe and has a sequence which can be partially or entirely complementary to a target DNA molecule to be detected, and one or more RNA bases are embedded in a complementary region of the probe and divide the complementary region of the probe into at least two segments, each of which independently has DNA bases and base substitutions that do not interfere with a hybridization of a nucleic acid strand that is less than or equal to 13 in total.

Ribonuclease HII (RNase HII) is an endoribonuclease that is suitable for cleaving the 5' end of a ribonucleic acid inside a double-stranded DNA, resulting in a 5' phosphate and a 3' hydroxyl end.

The term "base substitution" refers to a structure that contains no base, and connects the upstream and downstream of the nucleic acid strand to maintain the integrity of the probe as a whole, without interfering with the hybridization of the nucleic acid strand. For example, when a polymorphic site that does not need to be detected appears in a target sequence, a degeneracy analysis may be achieved by replacing the base in the probe at the corresponding position with a base substitution. The common base substitutions are a deoxynucleotide spacer (dSpacer) or a C3 spacer (Spacer). There may be 1, 2, 3, 4, 5, or 6 base substitutions.

There may be 1, 2, 3, 4, 5, 6, 7, 8, 9 or more RNA bases, which may be dispersed in DNA bases individually or in continuous fragments (each of the contiguous fragments contains at least two RNA bases), and further may be inserted fully and continuously into the middle of the probe.

In some embodiments, each segment has 1, 2, 3, 4, 5, 6, 7, 8, 9, 10, 11, or 12 DNA bases or base substitutions.

In some preferred embodiments, the RNA bases are distributed in the probe as discrete as possible to increase the efficiency of the cleaved product being dissociating into single strands.

In some preferred embodiments, RNase HII reacts at a temperature in a range from 55°C to 65°C, and the RNA bases are distributed in the probe at intervals of 3 to 8 bases.

In some embodiments, the probe includes only RNA bases and DNA bases.

In some embodiments, each segment has the DNA bases or base substitutions between 0 and 10.

In some embodiments, each segment has the DNA bases or base substitutions of less than or equal to 6.

The fragmentation of the probe may be detected by methods known to those skilled in the art, such as observing the size of the bands of a nucleic acid fragment by means of electrophoresis and the like. In some preferred embodiments, the probe is labeled with a label to determine whether the probe is cleaved.

In some embodiments, the probe has a Tm value expressed as a temperature that is greater than or equal to a value obtained by subtract 10°C from the reaction temperature of the cleavage reaction with the ribonuclease H II.

In some embodiments, at least two segments are labeled with different detectable identification elements, which are a specific nucleic acid sequence or a label labeled on bases and/or nucleic acid backbones. The label produces a detectable signal when the probe is cleaved at the RNA base with the ribonuclease H II.

In some embodiments, the label is labeled on bases of the probe at both ends.

In some embodiments, the label includes a fluorophore and a quencher, and at least one fluorophore and at least one quencher are located in different segments.

, The fluorophore will be separated from the quencher upon the cleavage of the probe, which results in an enhanced fluorescence, indicating the presence of a target nucleic acid. The fluorophore may include FAM, HEX, CY3, CY5, FITC, and the like, and the quencher may include BHQ1, BHQ2, BHQ3, TAMRA, Dabcyl, Dabsyl, and the like.

In some preferred embodiments, multiple modifications may be added on multiple RNA bases to achieve a reduced background and an enhanced signal. For example, "BHQ1-RNA base-FAM-RNA base-BHQ1" is labeled along the probe in 5' to 3' direction to reduce the negative background signal.

In other embodiments, the probe has an end fixed on the surface of a solid phase carrier, or bound to the solid phase carrier after the reaction is completed, and has the other end is coupled with a signal substance.

Whether the probe is cleaved is determined by the presence or absence of the signal substance.

The solid phase carrier may be a material, such as polystyrene, plastic, cellulose, polyacrylamide, polyethylene, polypropylene, cross-linked dextran, glass, silicone rubber, agarose gel, etc. The carrier may be in a form of test tubes, EP tubes, multiwell plates (especially ELISA plates), wells of microreaction plates, beads (especially magnetic beads), small discs, etc.

In some embodiments, the combination product further includes a test strip, wherein, the test strip includes a sample pad, a reaction membrane provided with a quality inspection region and a detection region along a liquid flow direction, and an absorption pad:
the label includes a first label and a second label;
the sample pad is coated with a signal substance, which is labeled with a first anti-label against the first label;
the quality inspection region is constantly coated with a second anti-label against the second label;
the detection region is constantly coated with a secondary antibody against the first anti-label;
the first label and the first anti-label, the second label and the second anti-label can form different label-anti-label complexes;
alternatively, the test strip comprises a sample pad, a reaction membrane provided with a quality inspection region and a detection region along a liquid flow direction, and an absorption pad;
the label includes a first label and a second label;
the sample pad is coated with a signal substance, which is labeled with a first anti-label against the first label;
the detection region is constantly coated with a second anti-label against the second label;
the quality inspection region is constantly coated with a secondary antibody against the first anti-label;
the first label and the first anti-label, the second label and the second anti-label can form different label-anti-label complexes.

"Secondary antibody" refers to an antibody against the first anti-label.

In some embodiments, the signal substance is selected from the group consisting of a fluorophore, a colorimetric label, a quantum dot, a colloidal gold, an alkyne group for Raman diffraction imaging, a cycloalkene for click reaction, a polymer-labeling initiating group, a polypeptide/protein molecule, an LNA/PNA, a non-natural amino acid and analog thereof, a non-natural nucleic acid and analog thereof, and a nanostructure;
the nanostructure includes an inorganic nanoparticle, a NV-center, an aggregation/assembly-induced luminescent molecule, a rare-earth ion ligand molecule, and a polyoxometalate.

The degradation products from the probe are detected by means of immunochromatography, and the integrity of the probe can be indicated by a signal substance such as colloidal gold or quantum dots.

In some embodiments, the combination of the label/anti-label in the label-anti-label complexes is selected from the group consisting of biotin or derivative thereof/streptavidin, biotin or derivative thereof/avidin, biotin or derivative thereof/neutravidin, hapten/antibody, antigen/antibody, receptor/ligand, digoxigenin/digoxigenin ligand, carbohydrate/agglutinin, and polynucleotide/complementary polynucleotide;
wherein the derivative of biotin is any one of D-biotin, activated biotin, biocytin, ethylenediamine biotin, cadaverine biotin and desthiobiotin; and
wherein the antigen and hapten may be a polypeptide, or protein or protein subunit, and such protein or protein subunit may also be an antibody or an antibody fragment.

The term "antibody" includes polyclonal antibodies and monoclonal antibodies. The term "antibody fragment" includes the antigen binding fragments of these antibodies, including Fab, F(ab')₂, Fd, Fv, scFv, bispecific antibodies and the minimum recognition unit of antibodies, as well as single-chain derivatives of these antibodies and fragments, for example, scFv-Fc, etc. The type of the antibody may select from IgG1, IgG2, IgG3, IgG4, IgA, IgM, IgE, or IgD. In addition, the term "antibody" includes naturally-occurring antibodies and non-naturally-occurring antibodies, including, for example, chimeric, bifunctional, humanized antibodies and human antibodies, and related synthetic isoforms thereof.

In some preferred embodiments, the nucleic acid sequences at the two flank of the RNA base of the probe are labeled with biotin and digoxigenin respectively, the gold nanoparticles are labeled with digoxigenin monoclonal antibody, and the streptavidin is immobilized on the nitrocellulose membrane. The probe is detected for its integrity by means of immunochromatography. The presence of the target leads to cleavage of the probe to be cut, and eventually the elimination of the colour on in the streptavidin line.

In some embodiments, the 3' end of the probe includes a non-extendable blocking moiety.

The blocked group may be selected from amino, phosphorylation, spacer, biotin, and other modification, and blockage may be achieved by labeling with a fluorophore, a quencher, etc., at the 3' end. It is also possible to extend the probe at one ends of the 5' and 3' ends, with introduced 3 to 8 base sequences that are reverse complementary to the other end, or extend the probe at both of the 5' and 3' ends to introduce 3 to 8 base sequences that are reverse complementary to the other end,, making the probe itself form a hairpin structure, and the two ends are labeled with a fluorophore and a quencher to reduce the negative background signal.

In some embodiments, blockage of the 3' end is achieved by introducing a nucleic acid sequence that is unrelated to the target sequence at the 3' end of the probe.

In some embodiments, the spacer modification is any one selected from ethylene glycol, C9 spacer (Spacer 9), C18 spacer (Spacer 18), dideoxy spacer [1', 2'-Dideoxyribose (dSpacer)], or C3 Spacer (Spacer 3).

In some embodiments, the spacer modification is the C3 spacer.

In some embodiments, the probe has one RNA base and has a length of less than 25 nt.

In some embodiments, the combination product further includes a LAMP primer, a NEAR primer, or an RPA primer.

According to a further aspect of the present disclosure, the present disclosure also relates to a kit including the combination product as described above.

In some embodiments, the kit further comprises a ribonuclease HII digestion buffer, a DNA polymerase, dNTPs, an amplification buffer and other components.

The present disclosure further relates to a use of the combination product as described above in the detection of SNP.

When used for SNP detection, the probe has one RNA base which is paired with the SNP site (paired with the wild type or mutant type).

RNase HII targets double-stranded DNA, which has certain requirements on the base pairing in the double-strand. The mismatched bases in the double-strand will affect the cleavage efficiency. The closer the mismatched bases are to the cleaving position, the lower the cleavage efficiency. When the RNA base position is mismatched, the cleavage efficiency is only about 1/10 of the level. Benefit from this and the presence of signal amplification in the reporting system, a single DNA single-stranded molecule may induce the cleavage of multiple probes, and the signal difference between the perfect match and the mismatch may be further increased by amplification, and eventual results may effectively distinguish differences between single bases and allows SNP detection.

In some embodiments, in SNP detection, the reaction temperature of the cleavage reaction with the ribonuclease H II is less than or equal to the temperature shown in the Tm value plus 10°C.

The system includes multiple probes for different alleles of the same SNP site. Each probe is labeled with a fluorophore corresponding to a different fluorescence channel, and with a quencher pairing with the fluorophore, which enables genotyping of samples in a single tube reaction.

The present disclosure also relates to a use of the combination product as described above in the detection of a target DNA obtained from amplification with a LAMP, NEAR or RPA primer.

The reporting system may follow the existing amplification method, to detect the end-point product. For example, NEAR, LAMP, RPA and other primers are directly added to the reaction system including RNase HII and probe for reaction and result determination. The reporting system, on the premise of being compatible with the amplification, may be directly incorporated into the amplification technology to identify and cleave the probe as the occurrence of the target nucleic acid, thereby achieving the fastest and easiest detection. The single-stranded target DNA produced by amplification may be directly detected, and for the system where the product is double-stranded DNA, the probe may be bond to the target sequence by denaturizing, annealing, etc., and thereby allows detection.

In some embodiments, the above applications specifically includes: 1. amplifying nucleic acid; 2. adding part or all of the reaction product to the RNase HII system after the reaction is completed, and melting the double-stranded DNA product by thermal denaturation, then followed by probe cleavage and detection.

In some embodiments, in the above applications, RNase HII and the probe are added to the amplification system, the amplification and probe cleavage are performed simultaneously, and then the probe is detected for its integrity. For example, the signal is detected in real time by fluorescence, or the end-point fluorescence value is determined after the reaction is completed, or the integrity of the probe is confirmed by means of immunochromatography and the like after the reaction is completed.

In some embodiments, in the above application, the reaction is carried out at a temperature in a range from 35°C to 45°C, and further preferably, the RNase HII derived from *E. coli* is used.

In some embodiments, the reaction temperature of the above application is in a range from 55°C to 65°C, using a thermophilic RNase, such as the RNase HII derived from *Thermus thermophilus or Pyrococcus abyssi.*

In some embodiments, each segment has the DNA bases or base substitutions of less than or equal to 6, and the amplification is performed at a temperature in a range from 35°C to 45°C (which may be 36°C, 37°C, 38°C, 39°C, 40°C, 41°C, 42°C, 43°C, or 44°C).

By shortening length of the DNA segment, the reporting system can operate at a lower reaction temperature, and can be used in lower temperature amplification systems such as RPA, and has a wide range of applications.

The principle provided by the present disclosure is as follows: RNase HII may specifically recognize the RNA bases in the DNA double-strand, and break the phosphodiester bond connecting the DNA bases in the 5' direction of the RNA bases, resulting in a nick in the DNA double-strand in the 5' direction of the RNA base. In organisms, DNA repair is triggered by this cleavage, which can remove RNA bases in the double-stranded DNA and ensure the accuracy for the genetic information. Based on the characteristic of RNase HII in strictly pairing with substrates, a single-stranded probe is designed in the present disclosure with one or more RNA bases embedded in the probe, and RNase HII is applied to the reporting system of the amplification system. Since RNase HII can only recognize and cleave double strands, it will not act on single-stranded probes. The probe will hybridize with a single-stranded DNA that is complementary thereto (if any) in the environment at a suitable temperature and be cleaved at a specific position by RNase HII. As the melting temperature of the cleaved probe drops, the stable binding with complementary single-stranded DNA cannot be kept and the probe will be dissociated from the complementary single-stranded DNA. The complementary single-stranded DNA may bind to a new probe and a new cycle starts to achieve signal amplification. In the process, the detection result is obtained by cleaving with RNase HII, which converts the input of single-stranded DNA complementary to the probe into the output of probe breakage, base-pairing to ensure the specificity of the generated product and to enable the signal amplification, and detecting the breakage of the probe.

The embodiments of the present disclosure will be described in detail below in combination with examples.

### Example 1 Effect of Tm value on reaction efficiency

The effect of probe length on the detection effect was tested at different reaction temperatures.

### 1. Design of primer and probe

**Table 1 Test primer and probe sequence for probe Tm value and reaction efficiency**

| Name | Sequence |
|---|---|
| Probe-Tm-59.5 | TGCACAATTTGCCCCCAGC |
| Probe-Tm-54.8 | GCACAATTTGCCCCCAG |
| Complement | GCTGGGGGCAAATTGTGCA |

In Table 1, Complement is the target sequence, Probe-Tm-59.5 and Probe-Tm-54.8 are two probes with different Tm values, have sequences which are both reverse complementary to Complement. In a direction from 5' to 3', the underlined bases indicated that they were modified, in which T in the Probe-Tm-59.5 probe was modified with a quencher, G was a RNA base, and C was modified with a fluorescent reporter group; T in the Probe-Tm-54.8 probe was modified with a quencher, G was a RNA base, and C was modified with a fluorescent reporter group.

### 2. Cleavage reaction

25 µL of reaction system: 20 mM Tris-HCl, 70 mM KCl, 10 mM (NH₄)₂SO₄, 0.1% Tween 20, 9 mM MgSO₄, 1 µg RNase HII, 0.02 µM Complement, 0.2 µM Probe-Tm -59.5 or Probe-Tm-54.8.

Reaction conditions: 65°C/60°C, 1 min, 35 cycles (fluorescence was collected every 1 min).

### 3. Results analysis

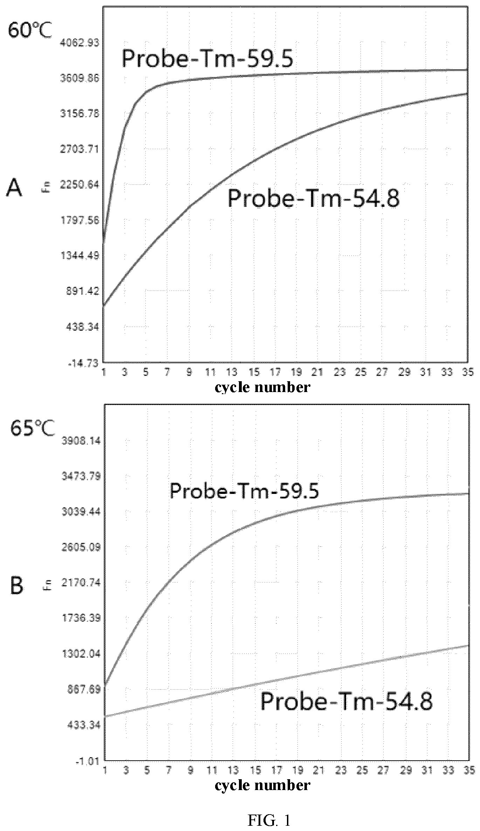

The result is shown in FIG. 1. At 60°C, both of the fluorescence from Probe-Tm-59.5 and Probe-Tm-54.8 grow relatively fast, and the fluorescence values from the two systems were relatively comparable to each other at 35 min. However, as the reaction temperature increased to 65°C, Probe-Tm-54.8 reacted at a significantly decreased rate, and the results showed that the probe Tm value is too much lower than the reaction temperature, which made it difficult to bind to the complementary template, resulting in only a small amount of fluorescence.

### Example 2 Effect of DNA base segment length on cleavage

A variety of probes were designed for a target sequence. The probes have the same basic sequences, except DNA base segment length separated by RNA bases. Probe: target sequence was added at a molar concentration of 100:1, and cleavage was performed in the presence/absence of DNA polymerase, respectively, to compare the effect of DNA base segment length on the cleavage.

### 1. Design of primer and probe

### Example 2 Test primer and probe sequences for effect of DNA base segment length on cleavage

| Name | Sequence |
|---|---|
| Probe-Segment ≤ 2 | ATCAAAGUTCUGCAGCUCTUAC |
| Probe-Segment ≤ 5 | ATCAAAGTTCUGCAGCUCTTAC |
| Probe-Segment ≤ 10 | ATCAAAGTTCTGCAGCUCTTAC |
| Probe-Segment ≤ 13 | ATCAAAGTTCTGCAGCTCTTAC |
| Probe-Segment < 15 | ATCAAAGTTCTGCAGCTCTTAC |
| Target sequence | GTAAGAGCTGCAGAACTTTGAT |

In Table 2, the probe-segments ≤ 2, ≤ 5, ≤ 10, ≤ 13, and ≤ 15 are five probes with different DNA base segment lengths separated by RNA bases, and have sequences which are all reverse complementary to the target sequence. In a direction from 5' to 3', the underlined bases indicated that they were modified, wherein A and U of probe-segment≤2 were RNA bases; A and U of probe-segment≤5 were RNA bases; A and U of probe-segment≤10 were RNA bases; A of probe-segment≤13 was RNA base; C of probe-segment≤15 was RNA base; in addition, the 5' ends of the five probes were all modified with a quencher, and the 3' ends were all modified with a fluorescent reporter group and a C3 spacer.

### 2. Cleavage reaction

25 µL reaction system: 20 mM Tris-HCl, 70 mM KCl, 10 mM (NH₄)₂SO₄, 0.1% Tween 20, 1.7 mM dNTPs, 9 mM MgSO₄, 8 U Bst 2.0 WarmStart, 1 µg RNase HII, 20 nM target sequence, five probes each at 200 nM: probe-segment≤2, probe-segment≤5, probe-segment≤10, probe-segment≤13, probe-segment≤15.

Reaction conditions: 60°C, 1 min, 60 cycles (fluorescence was collected every 1 min).

### 3. Result analysis

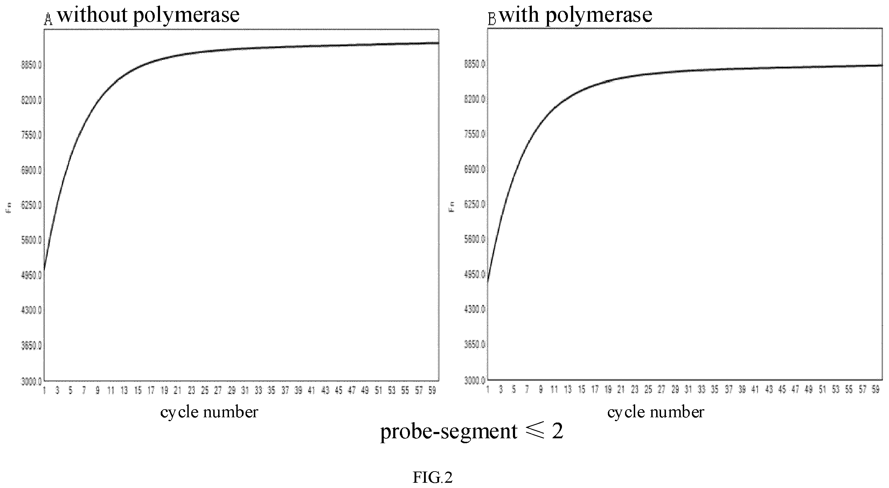

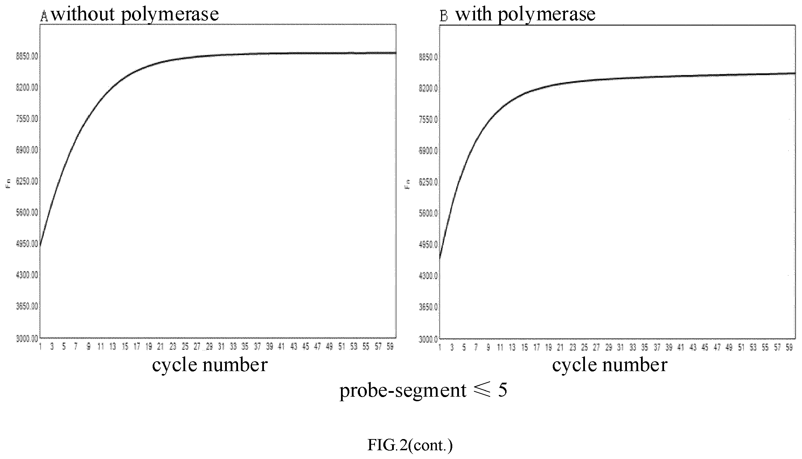

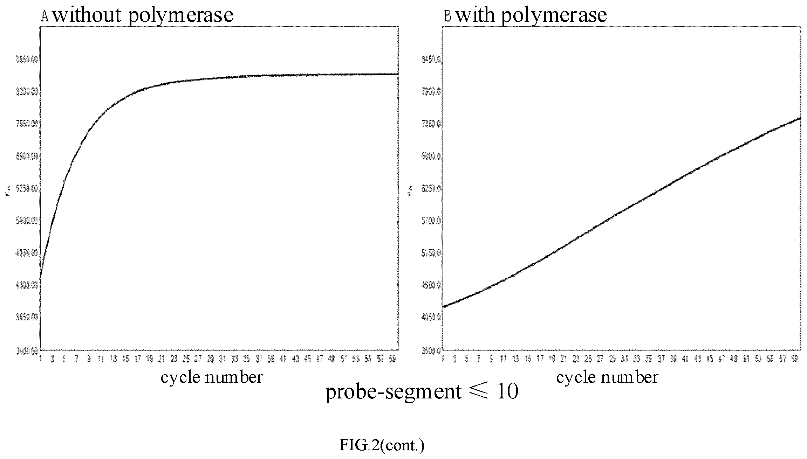

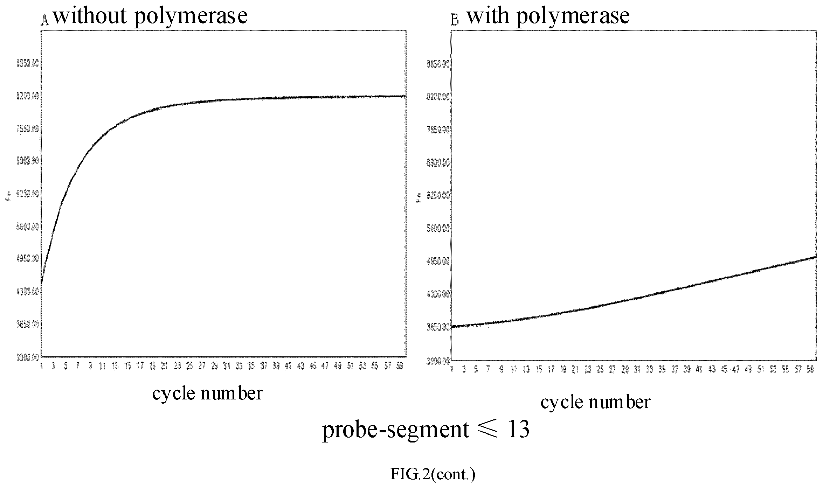

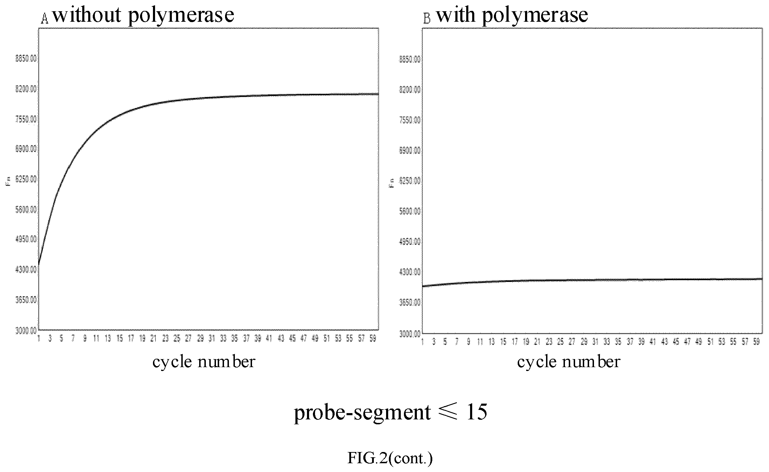

The result is shown in FIG. 2. When there was no DNA polymerase in the system, all probes showed a similar increasing trend, and the cleavage efficiencies of the probes were comparable. When DNA polymerase was present in the system, the fluorescence from probes with a DNA base segment length of 5 or less showed a similar increasing trend, and the probe-segment≤10 and probe-segment≤13 slowed increasing rates declined in sequence, but observable fluorescence signal increases, while the fluorescence increment of probe-segments ≤ 15 was significantly suppressed. This showed that upon identification and cleavage by RNase HII, the part of the probe upstream of the nick was used as a primer, and the target sequence was used as a template for extension under the action of DNA polymerase. The extension product existed stably in the form of double-stranded DNA. The single-stranded target sequence in the extension product could no longer activate further cleavage of probes. As the DNA base segment length shortens, the probe will be cleaved into shorter fragments after being recognized by RNase HII, resulting in a greater decrease in the Tm value of the product. The probe fragment could not remain in a double-stranded state but become in a free state, then the target sequence returned to the single-stranded DNA state, which could bind to a new probe and activate the reaction in the next round, promoting the signal amplification. Probes with a DNA base segment length of less than or equal to 13 all have the effect of increasing the signal, and those with a DNA base segment length of less than or equal to 5 have the best effect.

### Example 3 Effect of DNA base segment length on reportable temperature range

The effect of probes with different DNA base segment length on cleavage was compared at 37°C.

### 1. Design of primer and probe

**Table 3 Test primer and probe sequences for effect of DNA base segment length on reportable temperature range**

| Name | Sequence |
|---|---|
| N-probe-1R | AATTGCACAATTUGCCCCCAGCGCT |
| N-probe-3R | AATTGCACAATTUGCCCCCAGCGCT |
| Tm-645 | AGCGCTGGGGGCAAATTGTGCAATT |

In Table 3, Tm-645 is the target sequence, and N-probe-1R and N-probe-3R are probes with different DNA base segment lengths separated by RNA bases, and have sequences which are all reverse complementary to the target sequence. In a direction from 5' to 3', the underlined bases indicated that they were modified, wherein the 5' end of N-probe-1R was modified with a fluorophore, U was an RNA base, and the 3' end was modified with both quenching reporter group and C3 spacer; the 5' end of N-probe-3R was modified with a fluorophore, A, U, and A were RNA bases, and the 3' end was modified with both quenching reporter group and C3 spacer.

### 2. Cleavage reaction

25 µL of reaction system: 20 mM Tris-HCl, 70 mM KCl, 10 mM (NH₄)₂SO₄, 0.1% Tween 20, 1 µg RNase HII, 20 nM Tm-645, 200 nM N-probe-1R or N-probe-3R.

Reaction conditions: 37°C, 1 min, 15 cycles (fluorescence was collected every 1 min).

### 4. Result analysis

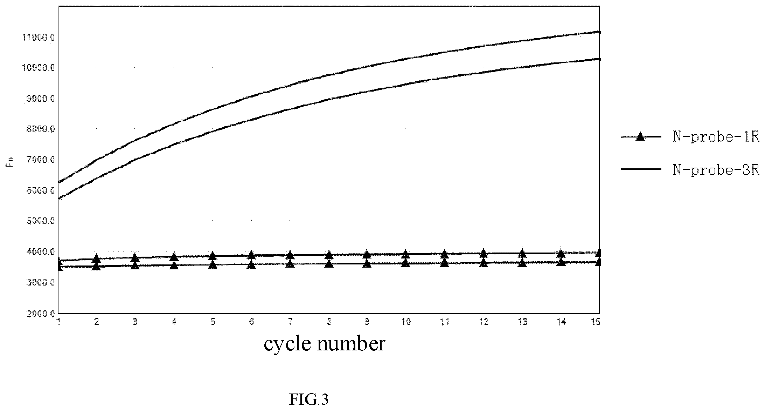

The result is shown in FIG. 3. The N-probe-1R has a DNA segment length of 12, and the N-probe-3R has a maximum DNA segment length of 6. The fluorescence of N-probe-3R probe still had an increasing trend at 37°C, while the cleavage effect of N-probe-1R probe was inhibited. This shows that shortening the DNA base segment length of the probe could broaden the reportable temperature range, so that the probe works normally at lower reaction temperatures. The probe with the maximum DNA segment length of 6 could work in the system at 37°C, and could effectively adapt to reaction systems such as RPA that perform amplification at lower temperatures.

### Example 4 Test for specificity of the reporting system - real-time fluorescence detection for SARS-CoV-2

The target was amplified by LAMP with a set of six primers, and the generation of specific products was reported in real time with RNase HII and probes in the system.

### 1. Design of primer and probe

**Table 4 Test primer and probe sequences for specificity of real-time fluorescence detection reporting system for SARS-CoV-2**

| Name | Sequence |
|---|---|
| SARS-CoV-2-B3 | GCGTCAATATGCTTATTCAGC |
| SARS-CoV-2-BIP | TCAGCGTTCTTCGGAATGTCGCTGTG TAGGTCAACCACG |
| SARS-CoV-2-LB | TGGCATGGAAGTCACACC |
| SARS-CoV-2-F3 | AACACAAGCTTTCGGCAG |
| SARS-CoV-2-FIP | TGCGGCCAATGTTTGTAATCAGCCAA GGAAATTTTGGGGAC |
| SARS-CoV-2-LF | TTCCTTGTCTGATTAGTTC |
| SARS-CoV-2-Probe-F | AGCGCTGGGGGCAAATTGTGCAATT |

In Table 4, SARS-CoV-2-B3, SARS-CoV-2-BIP, SARS-CoV-2-LB, SARS-CoV-2-F3, SARS-CoV-2-FIP, SARS-CoV-2-LF were amplification primers for SARS-CoV-2, and SARS-CoV-2-Probe-F was a detection probe for SARS-CoV-2. In a direction from 5' to 3', the underlined bases indicated that they were modified, T of the probe SARS-CoV-2-Probe-F was modified a quencher, A was an RNA base, C was modified with a reporter fluorophore, and the 3' end was modified with a C3 spacer.

### 2. Isothermal amplification reaction

25 µL of reaction system: 20 mM Tris-HCl, 70 mM KCl, 10 mM (NH₄)₂SO₄, 0.1% Tween 20, 9 mM MgSO₄, 1.7 mM dNTPs, 8 U Bst 2.0 WarmStart, 1.6 µM SARS- CoV-2-BIP/SARS-CoV-2-FIP, and 0.4 µM SARS-CoV-2-LB/SARS-CoV-2-LF/SARS-CoV-2-B3/SARS-CoV-2-F3. The detection system of RNase HII further included 1 µg of RNase HII, and 0.2 µM of SARS-CoV-2-Probe-F, and the system for the dye method further included 0.5 × LAMP fluorescent dye.

Reaction conditions: 63°C, 1 min, 40 cycles (fluorescence was collected every 1 min).

### 3. Results analysis

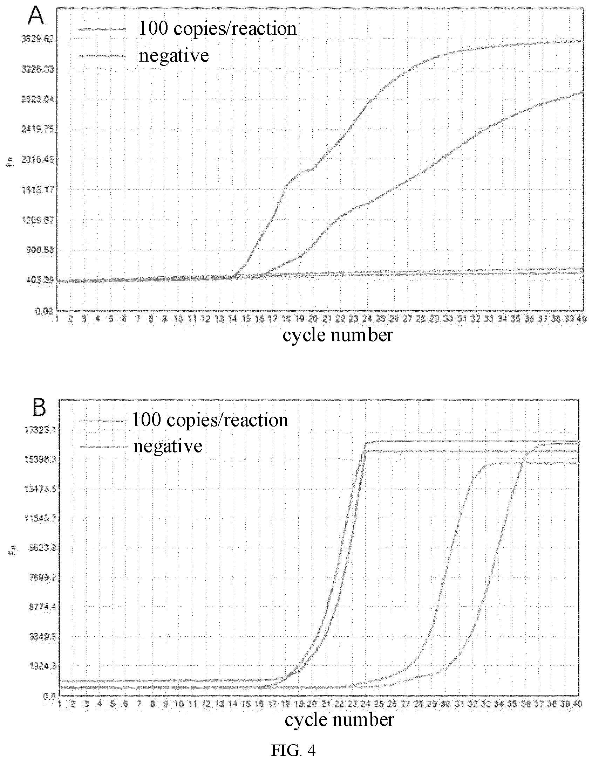

The result is shown in FIG. 4. When the dye was used for indication, non-specific signal could generate in the negative control; however RNase HII and the probe as the reporting system could ensure that no non-specific signal generates in the negative control, with high specificity.

### Example 5 Test for reporting system amplification effect - real-time fluorescence detection for SARS-CoV-2

For the same set of LAMP amplification primers, probes with the same target sequence but different DNA segment lengths were designed respectively, and compared for effect of segment length on the reaction sensitivity was.

### 1. Design of primer and probe

**Table 5 Test primer and probe sequences for amplification effect of real-time fluorescence detection reporting system for SARS-CoV-2**

| Name | Sequence |
|---|---|
| SARS-CoV-2-B3 | GCGTCAATATGCTTATTCAGC |
| SARS-CoV-2-BIP | TCAGCGTTCTTCGGAATGTCGCTGTGT AGGTCAACCACG |
| SARS-CoV-2-LB | TGGCATGGAAGTCACACC |
| SARS-CoV-2-F3 | AACACAAGCTTTCGGCAG |
| SARS-CoV-2-FIP | TGCGGCCAATGTTTGTAATCAGCCAA GGAAATTTTGGGGAC |
| SARS-CoV-2-LF | TTCCTTGTCTGATTAGTTC |
| SARS-CoV-2-Probe-12 | AGCGCTGGGGGCAAATTGTGCAATT |
| SARS-CoV-2-Probe-6 | AGCGCTGGGGGCAAATTGTGCAATT |

In Table 5, SARS-CoV-2-B3, SARS-CoV-2-BIP, SARS-CoV-2-LB, SARS-CoV-2-F3, SARS-CoV-2-FIP, SARS-CoV-2-LF were amplification primers for SARS-CoV-2, and SARS-CoV-2-Probe-12 and SARS-CoV-2-Probe-6 were detection probes for SARS-CoV-2. In a direction from 5' to 3', the underlined bases indicated that they were modified, wherein the 5' end of the probe SARS-CoV-2-Probe-12 was modified with a quencher group, A was an RNA base, and the 3' end was modified with a reporter fluorophore and C3 spacer; the 5' end of SARS-CoV-2-Probe-6 was modified with a quencher, G, A, and G were RNA bases, and the 3' end was modified with a reporter fluorophore and C3 spacer.

### 2. Isothermal amplification reaction

25 µL of reaction system: 20 mM Tris-HCl, 70 mM KCl, 10 mM (NH₄)₂SO₄, 0.1% Tween 20, 9 mM MgSO₄, 1.7 mM dNTPs, 8 U Bst 2.0 WarmStart, 1.6 µM SARS- CoV-2-BIP/SARS-CoV-2-FIP, and 0.4 µM SARS-CoV-2-LB/SARS-CoV-2-LF/SARS-CoV-2-B3/SARS-CoV-2-F3. The detection system of RNase HII further included 1 µg of RNase HII, and 0.2 µM of SARS-CoV-2-Probe-12 or SARS-CoV-2-Probe-6.

Reaction conditions: 63°C, 1 min, 60 cycles (fluorescence was collected every 1 min).

### 3. Results analysis

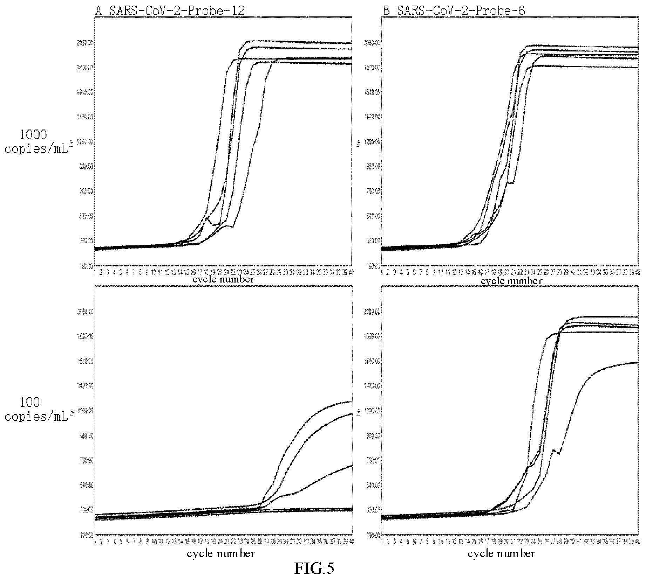

The result is shown in FIG. 5. Two probes with the maximum spacer segment of 12 and 6 bases were used to detect plasmid DNA at different concentrations with the same primer combination. The plasmids could be stably detected at only 1000 copies/mL in SARS-CoV-2-Probe-12 probe group, but at 100 copies/mL in SARS-CoV-2-Probe-6 probe group. Therefore, shortening the DNA segment length, when used in combination with the amplification reaction, facilitates the dissociation of the cleaved productand has the beneficial effect of improving the sensitivity of the method.

### Example 6 Application example of reporting system - colloidal gold detection (line display method) for SARS-CoV-2.

The target was amplified by LAMP with a set of six primers, and the probe was cleaved by the RNase HII in the system synchronously as the product increased. After the LAMP reaction, if there was no target, the probe remained intact, and if there was a target, the probe was cleaved. In the colloidal gold test strip, the gold conjugate pad contained digoxigenin antibody-labelled gold nanoparticles, streptavidin was immobilized in the quality control line, and the secondary antibody was immobilized in the detection line. When the probe was intact, the antibody and streptavidin of the gold nanoparticles recognized the two labels in the probe respectively, and thereby forms a sandwich therewith, so that the gold nanoparticles were captured in the quality control line for color development. At the same time, since most of the gold nanoparticles were captured in the quality control line, the detection line did not develop; when the probe was cleaved, streptavidin could capture only the cleaved halves of-probe, but no gold nanoparticles. The more cleaved probes, the less gold nanoparticles captured by the quality control line, the more gold nanoparticles flowing to the detection line and captured by the secondary antibody, and the darker the color of the detection line. The detection line developed in the presence of the target sequence, but did not develop in the absence of the target sequence.

### 1. Design of primer and probe

**Table 6 Primer and probe sequences for colloidal gold detection (line display method) for SARS-CoV-2**

| Name | Sequence |
|---|---|
| SARS-CoV-2-B3 | GCGTCAATATGCTTATTCAGC |
| SARS-CoV-2-BIP | TCAGCGTTCTTCGGAATGTCGCTGTG TAGGTCAACCACG |
| SARS-CoV-2-LB | TGGCATGGAAGTCACACC |
| SARS-CoV-2-F3 | AACACAAGCTTTCGGCAG |
| SARS-CoV-2-FIP | TGCGGCCAATGTTTGTAATCAGCCAA GGAAATTTTGGGGAC |
| SARS-CoV-2-LF | TTCCTTGTCTGATTAGTTC |
| SARS-CoV-2-Probe-LFB | AGCGCTGGGGGCAAATTGTGCAATT |

In Table 6, SARS-CoV-2-B3, SARS-CoV-2-BIP, SARS-CoV-2-LB, SARS-CoV-2-F3, SARS-CoV-2-FIP, SARS-CoV-2-LF were amplification primers for SARS-CoV-2, and SARS-CoV-2-Probe-LFB was a detection probe for SARS-CoV-2. In a direction from 5' to 3', the underlined bases indicated that they were modified, the 5' end of the probe SARS-CoV-2-Probe-LFB was modified with digoxigenin, A was an RNA base, and the 3' end was modified with biotin.

### 2. Isothermal amplification reaction

25 µL of reaction system: 20 mM Tris-HCl, 70 mM KCl, 10 mM (NH₄)₂SO₄, 0.1% Tween 20, 9 mM MgSO₄, 1.7 mM dNTP, 8 U Bst 2.0 WarmStart, 1 µg RNase HII, 1.6 µM SARS-CoV-2-BIP/SARS-CoV-2-FIP, 0.4 µM SARS-CoV-2-LB/SARS-CoV-2-LF/SARS-CoV-2-B3/SARS-CoV-2 -F3, 0.2 µM SARS-CoV-2-Probe-LFB.

Reaction conditions: 63°C, 30 min.

### 3. Colloidal gold detection

The sample pad of a test strip was inserted at an end into an EP tube containing the amplification product. The chromatography was carried out for 10 minutes before interpretation of the result.

### 4. Result analysis

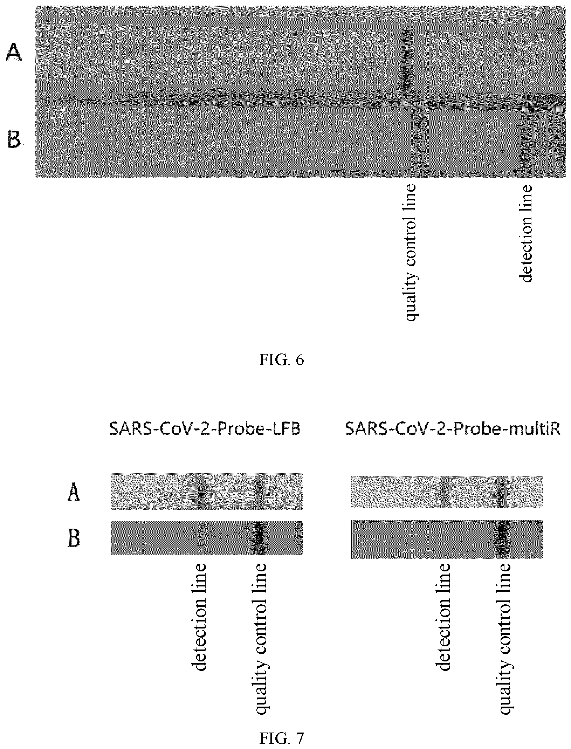

The result is shown in FIG. 6. In the negative control, the probe remained intact, and the antibody-labeled gold nanoparticles-probe-streptavidin formed as a sandwich. The gold nanoparticles were captured on the detection line, resulting in a color-developing detection line. In the detection of the SARS-CoV-2 sample, probes were cleaved as the reaction proceeded, so the gold nanoparticles could not be captured on the detection line, resulting in no color-developing detection line.

### Example 7 Application example of reporting system - colloidal gold detection (line elimination method) for SARS-CoV-2

The target was amplified by LAMP with a set of six primers, and the probe was cleaved by the RNase HII in the system synchronously as the product increased. After the LAMP reaction, if there was no target, the probe remained intact, and if there was a target, the probe was cleaved. In the colloidal gold test strip, the gold conjugate pad contained digoxigenin antibody-labelled gold nanoparticles, streptavidin was immobilized in the detection line, and the secondary antibody was immobilized in the quality control line. When the probe was intact, the antibody and streptavidin on the gold nanoparticles recognized the two labels of the probe respectively, and thereby forms a sandwich therewith, so that the gold nanoparticles were captured in the detection line for color development; when the probe was cleaved, streptavidin could capture only the cleaved halves of probe, but no gold nanoparticles, thus the detection line did not develop. The detection line developed in the absence of the target sequence, and disappeared in the presence of the target sequence. The gold conjugate pad had an excess amount of gold nanoparticles immobilized thereon. Whether or not the detection line capture the gold nanoparticles, surplus gold nanoparticles could be combined with the secondary antibody in the quality control line for color development, to confirm that the test strip functions normally in the reaction.

In the examples, two probes, i.e. SARS-CoV-2-Probe-LFB and SARS-CoV-2-Probe-multiR, were used respectively. The former had a single RNA base, and the latter had multi-RNA bases divided the probe into shorter spaced DNA segments, to compare the benefits of the short segment design.

### 1. Design of primer and probe

**Table 7 Primer and probe sequences for colloidal gold detection (line elimination method) for SARS-CoV-2 [0140]**

| Name | Sequence |
|---|---|
| SARS-CoV-2-B3 | GCGTCAATATGCTTATTCAGC |
| SARS-CoV-2-BIP | TCAGCGTTCTTCGGAATGTCGCTGTG TAGGTCAACCACG |
| SARS-CoV-2-LB | TGGCATGGAAGTCACACC |
| SARS-CoV-2-F3 | AACACAAGCTTTCGGCAG |
| SARS-CoV-2-FIP | TGCGGCCAATGTTTGTAATCAGCCAA GGAAATTTTGGGGAC |
| SARS-CoV-2-LF | TTCCTTGTCTGATTAGTTC |
| SARS-CoV-2-Probe-LFB | AGCGCTGGGGGCAAATTGTGCAATT |
| SARS-CoV-2-Probe-multiR | AGCGCUGGGGGCAAATTGUGCAATT |

In Table 7, SARS-CoV-2-B3, SARS-CoV-2-BIP, SARS-CoV-2-LB, SARS-CoV-2-F3, SARS-CoV-2-FIP, SARS-CoV-2-LF were amplification primers for SARS-CoV-2, and SARS-CoV-2-Probe-LFB and SARS-CoV-2-Probe-multiR were detection probes for SARS-CoV-2. In a direction from 5' to 3', the underlined bases indicated that they were modified, wherein the 5' end of the probe SARS-CoV-2-Probe-LFB was modified with digoxigenin, C was an RNA base, and the 3' end was modified with biotin; the 5' end of the SARS-CoV-2-Probe-multiR probe was modified with digoxigenin, U, C and U were RNA bases, and the 3' end was modified with biotin.

### 2. Isothermal amplification reaction

25 µL of reaction system: 20 mM Tris-HCl, 70 mM KCl, 10 mM (NH₄)₂SO₄, 0.1% Tween 20, 1.7 mM dNTP, 9 mM MgSO₄, 8 U Bst 2.0 WarmStart, 1 µg RNase HII, 1.6 µM SARS-CoV-2-BIP/SARS-CoV-2-FIP, 0.4 µM SARS-CoV-2-LB/SARS-CoV-2-LF/SARS-CoV-2-B3/SARS-CoV-2 -F3, 20 nM SARS-CoV-2-Probe-LFB or SARS-CoV-2-Probe-multiR.

Reaction conditions: 63°C, 30 min.

### 3. Colloidal gold detection

The sample pad of a test strip was inserted at an end into an EP tube containing the amplification product. The chromatography was carried out for 10 minutes before interpretation of the result.

### 4. Result analysis

The result is shown in FIG. 7. In the test group using the single-RNA base probe SARS-CoV-2-Probe-LFB, although the cleavage of the probe could be triggered in the positive samples, this cleavage was not sufficient, resulting in the color of the detection line fade but not completely disappear; in the SARS-CoV-2-Probe-multiR test group, the detection line completely disappeared in the result from the positive sample, which would not cause misinterpretation of the results.

### Example 8 Effect of probe length on single base recognition

The recognition effect of single base changes in target nucleic acid was tested with different probe lengths.

**Table 8 Test primer and probe sequences for effect of probe length on single base recognition**

| Name | Sequence |
|---|---|
| Probe-Tm-59.5 | TGCACAATTTGCCCCCAGC |
| Probe-extended | AATTGCACAATTTGCCCCCAGCGCT |
| Complement-25 | AGCGCTGGGGGCAAATTGTGCAATT |
| Mism | AGCGCTGGGGGTAAATTGTGCAATT |

In Table 8, Complement-25 and Mism were target sequences which were reverse complementary to the two probes, but the base in Mism corresponding to the probe RNA base G was T; Probe-Tm-59.5 and Probe-extended were two probes with different lengths, and have sequences which were reverse complementary to the target sequence, respectively. In a direction from 5' to 3', the underlined bases indicated that they were modified, T of the Probe-Tm-59.5 probe was modified with a quencher, G was RNA bases, and C was modified with a fluorescent reporter group; T of the Probe-extended probe was modified with a quencher, G was RNA bases, and C was modified with a fluorescent reporter group.

### 2. Cleavage reaction

25 µL of reaction system: 20 mM Tris-HCl, 70 mM KCl, 10 mM (NH₄)₂SO₄, 0.1% Tween 20, 9 mM MgSO₄, 1 µg RNase HII, 0.02 µM Complement or Mism, and 0.2 µM Probe -Tm-59.5 or Probe-extended.

Reaction conditions: 60°C, 1 min, 60 cycles (fluorescence was collected every 1 min).

### 3. Results analysis

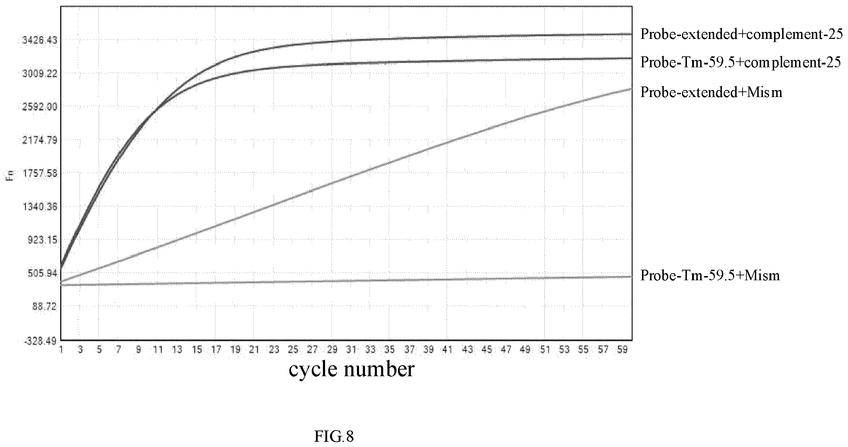

The result is shown in FIG. 8. Probe-Tm-59.5 could accurately distinguish Complement-25 from Mism. The fluorescence will increase only in the case of complete complementarity. The single base mutation would cause the failure of probe cleavage, thus the reporting system has stronger specificity and could be used for detection of gene mutation. Probe-extended would generate intense fluorescence when identifying Complement-25, but also intense fluorescence a certain amount of fluorescence when identifying Mism, which could lead to false positive result. Therefore, probe having a length of greater than or equal to 25 bases could lead to non-specific cleavage during SNP analysis, resulting in inaccurate results.

### Example 9 Application of the reporting system to SNP detection -real-time fluorescence detection for SNP site at rs1815739

The target was amplified by LAMP with a set of six primers, and the genotype of the SNP site was reported in real time through RNase HII and probes in the system. The reaction system included both of two probes targeting two alleles at the SNP site and labelled with two different fluorophores respectively. The base at SNP site of the target sequence could only trigger the cleavage of the perfectly paired probe, to produce fluorescence corresponding to the channel. The genotype of the sample could be obtained by determining whether the fluorescence generates in the two channels.

### 1. Design of primer and probe

**Table 9 Primer and probe sequences for real-time fluorescence detection for rs1815739**

| Name | Sequence |
|---|---|
| 181-F3 | TGCTGCCCTTTCTGTTGC |
| 181-B3 | TCCCACTTGGTGTTGATGTC |
| 181-FIP | TGATCGTGCGCTGTCAGCAGGGGACACCAG CTGACACT |
| 181-BIP | GTGCCATCATGGGCATCCAGGTGTAGGGATT GGTGGAGCAG |
| 181-LF | AGGCTCTGGGGACGACA |
| 181-LB | GGGTGAGATCCAGAAGATCTGC |
| 181-C | CTCGCTCTCGGTCAGCCTC |
| 181-T | CTCGCTCTCAGTCAGCCTC |

In Table 9, 181-F3, 181-B3, 181-FIP, 181-BIP, 181-LF and 181-LB were amplification primers for the rs1815739 site, 181-C was a detection probe for the allele C, and 181- T was a detection probe for the T allele. In a direction from 5' to 3', the underlined bases indicated that they were modified, wherein C of the 181-C probe was modified with a reporter fluorescent HEX group, G was an RNA base, T was modified with a quencher, and the 3' end was modified with C3 spacer; C of 181-T probe was modified with a reporter fluorescent FAM group, A was an RNA base, T was modified with a quencher, and the 3' end was modified with C3 spacer.

### 2. Isothermal amplification reaction

25 µL of reaction system: 20 mM Tris-HCl, 70 mM KCl, 10 mM (NH₄)₂SO₄, 0.1% Tween 20, 1.7 mM dNTP, 9 mM MgSO₄, 8 U Bst 2.0 WarmStart, 1 µg RNase HII, 1.6 µM 181-BIP/181-FIP, 0.4 µM 181-LB/181-LF/181-B3/181-F3, 0.2 µM 181-C/181-T.

Reaction conditions: 63°C, 1 min, 40 cycles (fluorescence was collected every 1 min).

### 3. Results analysis

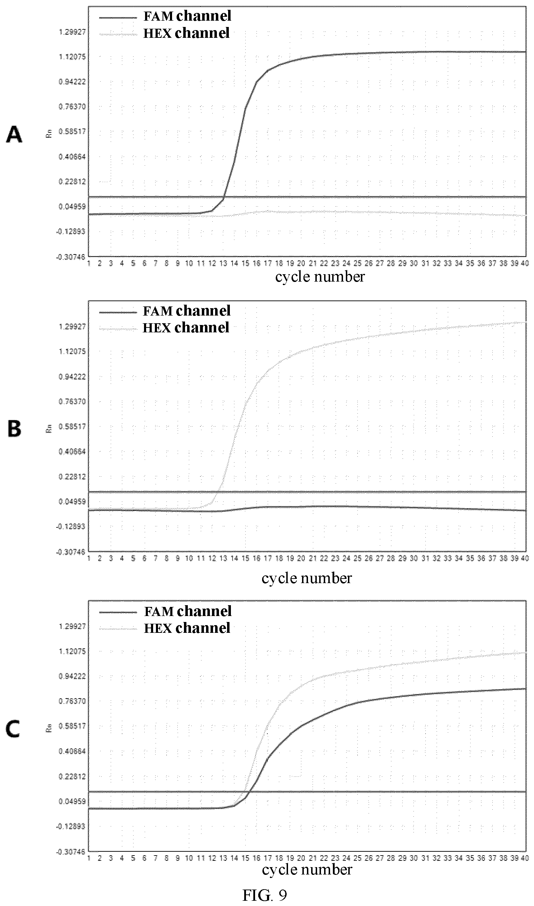

The result is shown in FIG. 9. The genotype of the sample could be accurately determined through the fluorescent channel with signal.

### Example 10 Example of applying reporting system to SNP detection -colloidal gold detection (line display method) of SNP site rs1815739

The target was amplified by LAMP with a set of six primers, and the probe for the genotype at the corresponding SNP site was cleaved by RNase HII in the system as the amplification product increased. In the presence of the target sequence, bases at the SNP site could trigger the cleavage of the perfectly paired probe. Two probes were designed to target two genes at the SNP site respectively. The probes were modified with labels that could be recognized by colloidal gold test strips. The genotype in the sample could be determined by the color development in the two colloidal gold test strips. The probe would be cleaved and the detection line in the test strip would develop, in the presence of perfectly paired gene at the SNP site in the target sequence; the probe would not be cleaved, the quality control line rather than the detection line in the test strip would develop, in the absence of paired gene at the SNP site.

### 1. Design of primer and probe

**Table 10 Primer and probe sequences for colloidal gold detection (line display method) for rs1815739**

| Name | Sequence |
|---|---|
| 181-F3 | TGCTGCCCTTTCTGTTGC |
| 181-B3 | TCCCACTTGGTGTTGATGTC |
| 181-FIP | TGATCGTGCGCTGTCAGCAGGGACAC CAGCTGACACT |
| 181-BIP | GTGCCATCATGGGCATCCAGGTGTAGG GATTGGTGGAGCAG |
| 181-LF | AGGCTCTGGGGACGACA |
| 181-LB | GGGTGAGATCCAGAAGATCTGC |
| 181-C-LFB | CTCGCTCTCGGTCAGCCTC |
| 181-T-LFB | CTCGCTCTCAGTCAGCCTC |

In Table 10, 181-F3, 181-B3, 181-FIP, 181-BIP, 181-LF and 181-LB were amplification primers for the rs1815739 site, 181-C-LFB was a detection probe for the allele C, and 181-T-LFB was a detection probe for the T allele. In a direction from 5' to 3', the underlined bases indicated that they were modified, wherein the 5' end of the 181-C-LFB probe was modified with digoxigenin, C was an RNA base, and the 3' end was modified with biotin; the 5' end of the 181-T-LFB probe was modified with digoxigenin, A was an RNA base, and the 3' end was modified with biotin.

### 2. Isothermal amplification reaction

25 µL of reaction system: 20 mM Tris-HCl, 70 mM KCl, 10 mM (NH₄)₂SO₄, 0.1% Tween 20, 1.7 mM dNTP, 9 mM MgSO₄, 8 U Bst 2.0 WarmStart, 1 µg RNase HII, 1.6 µM 181-BIP/181-FIP, 0.4 µM 181-LB/181-LF/181-B3/181-F3, 0.2 µM 181-C-LFB/181-T-LFB.

Reaction conditions: 63°C, 1 min, 40 cycles (fluorescence was collected every 1 min).

### 3. Colloidal gold detection

The sample pad of a test strip was inserted at an end into an EP tube containing the amplification product. The chromatography was carried out for 10 minutes before interpretation of the result.

### 4. Result analysis

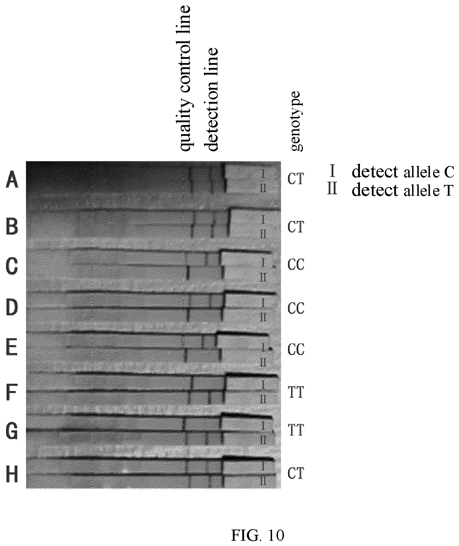

The result is shown in FIG. 10. By observing the color development in the two colloidal gold test strips that detecting the cleavage of probes for sample genotypes C and T, respectively, the sample genotype could be accurately determined.

Each of the technical features of the above-mentioned embodiments may be combined arbitrarily. To simplify the description, not all the possible combinations of each of the technical features in the above embodiments are described. However, all of the combinations of these technical features should be considered as within the scope of this disclosure, as long as such combinations do not contradict with each other.

## Claims

1. A combination product for DNA detection, comprising a ribonuclease H II and a probe,
wherein the probe is a single-stranded probe and has a sequence which is partially or entirely complementary to a target DNA molecule to be detected, and at least two RNA bases are embedded in the complementary region of the probe and divide the complementary region of the probe into at least three segments, each of which independently has 1, 2, 3, 4, 5 or 6 DNA bases and base substitutions that do not interfere with a hybridization of a nucleic acid strand in total, and wherein the RNA bases are distributed discretely in the probe.

2. The combination product according to claim 1, wherein the probe has a Tm value expressed as a temperature that is greater than or equal to a value obtained by subtract 10°C from the reaction temperature of the cleavage reaction with the ribonuclease H II, wherein the reaction temperature of the cleavage reaction with the ribonuclease HII is in a range from 55°C-65°C.

3. The combination product according to claim 1, wherein at least two segments are labeled with different detectable identification elements, which are a specific nucleic acid sequence or a label labeled on bases and/or nucleic acid backbones; and the label produces a detectable signal when the probe is cleaved at the RNA base with the ribonuclease H II.

4. The combination product according to claim 3, wherein the label comprises a fluorophore and a quencher, and at least one fluorophore and at least one quencher are located in different segments.

5. The combination product according to claim 3, wherein the probe has an end fixed on a surface of a solid phase carrier, or bound to the solid phase carrier upon reaction, and the other end coupled with a signal substance.

6. The combination product according to claim 3, further comprising a test strip,
wherein, the test strip comprises a sample pad, a reaction membrane provided with a quality inspection region and a detection region along a liquid flow direction, and an absorption pad;
the label comprises a first label and a second label;
the sample pad is coated with a signal substance, which is labeled with a first anti-label against the first label;
the quality inspection region is constantly coated with a second anti-label against the second label;
the detection region is constantly coated with a secondary antibody against the first anti-label;
the first label and the first anti-label, as well as the second label and the second anti-label, form different label-anti-label complexes;
or, the test strip comprises a sample pad, a reaction membrane provided with a quality inspection region and a detection region along a liquid flow direction, and an absorption pad;
the label comprises a first label and a second label;
the sample pad is coated with a signal substance, which is labeled with a first anti-label against the first label;
the detection region is constantly coated with a second anti-label against the second label;
the quality inspection region is constantly coated with a secondary antibody against the first anti-label;
the first label and the first anti-label, as well as the second label and the second anti-label, form different label-anti-label complexes.

7. The combination product according to claim 6, wherein the signal substance is selected from the group consisting of a fluorophore, a colorimetric label, a quantum dot, a colloidal gold, an alkyne group for Raman diffraction imaging, a cycloalkene for click reaction, a polymer-labeling initiating group, a polypeptide/protein molecule, an LNA/PNA, a non-natural amino acid and analog thereof, a non-natural nucleic acid and analog thereof, and a nanostructure;
the nanostructure comprises an inorganic nanoparticle, a NV-center, an aggregation/assembly-induced luminescent molecule, a rare-earth ion ligand molecule, and a polyoxometalate.

8. The combination product according to claim 6, wherein the combination of the label/anti-label in the label-anti-label complexes is selected from the group consisting of biotin or derivative thereof/streptavidin, biotin or derivative thereof/avidin, biotin or derivative thereof/neutravidin, hapten/antibody, antigen/antibody, receptor/ligand, digoxigenin/digoxigenin ligand, carbohydrate/agglutinin, and polynucleotide/complementary polynucleotide;
wherein the derivative of biotin is any one of D-biotin, activated biotin, biocytin, ethylenediamine biotin, cadaverine biotin or desthiobiotin.

9. The combination product according to claim 1, wherein the probe has a 3' end comprising a non-extendable blocking moiety.

10. The combination product according to any one of claims 1 to 9, wherein the probe has two RNA bases, and the complementary region of the probe has a length of less than 25 nt.

11. The combination product according to any one of claims 1 to 9, further comprising a LAMP primer, a NEAR primer or an RPA primer.

12. A kit comprising the combination product of any one of claims 1 to 11.

13. Use of the combination product of claim 10 in the detection for SNP, wherein the probe has two RNA bases.

14. The use according to claim 13, wherein in the detection for SNP, a reaction temperature of a cleavage reaction with the ribonuclease H II is less than or equal to a temperature shown in a Tm value plus 10°C, wherein the reaction temperature of the cleavage reaction with the ribonuclease H II is in a range from 55°C-65°C.

15. Use of the combination product of any one of claims 1 to 9 in the detection of a target DNA obtained from an amplification with a LAMP, NEAR or RPA primer.

16. The use according to claim 15, wherein each segment has of the DNA bases or base substitutes of less than or equal to 6, and the amplification is performed at a temperature of 35°C to 45°C.

## Patentansprüche

1. Kombinationsprodukt zum DNA-Nachweis, umfassend eine Ribonuklease H II und eine Sonde,
wobei die Sonde eine einzelsträngige Sonde ist und eine Sequenz aufweist, die teilweise oder vollständig komplementär zu einem nachzuweisenden Ziel-DNA-Molekül ist, und wenigstens zwei RNA-Basen in die komplementäre Region der Sonde eingebettet sind und die komplementäre Region der Sonde in wenigstens drei Segmente unterteilen, von denen jedes unabhängig 1, 2, 3, 4, 5 oder 6 DNA-Basen und Basensubstitutionen aufweist, die eine Hybridisierung eines Nukleinsäurestrangs insgesamt nicht stören, und wobei die RNA-Basen diskret in der Sonde verteilt sind.

2. Kombinationsprodukt nach Anspruch 1, wobei die Sonde einen Tm-Wert aufweist, ausgedrückt als eine Temperatur, die größer oder gleich einem Wert ist, der durch Subtraktion von 10 °C von der Reaktionstemperatur der Spaltreaktion mit der Ribonuklease H II erhalten wird, wobei die Reaktionstemperatur der Spaltreaktion mit der Ribonuklease HII in einem Bereich von 55 °C-65 °C liegt.

3. Kombinationsprodukt nach Anspruch 1, wobei wenigstens zwei Segmente mit unterschiedlichen nachweisbaren Identifizierungselementen markiert sind, die eine spezifische Nukleinsäuresequenz oder eine Markierung sind, die auf Basen und/oder Nukleinsäurerückgraten markiert ist; und die Markierung ein nachweisbares Signal erzeugt, wenn die Sonde an der RNA-Base mit der Ribonuklease HII gespalten wird.

4. Kombinationsprodukt nach Anspruch 3, wobei die Markierung ein Fluorophor und einen Quencher umfasst und sich wenigstens ein Fluorophor und wenigstens ein Quencher in verschiedenen Segmenten befinden.

5. Kombinationsprodukt nach Anspruch 3, wobei die Sonde ein auf einer Oberfläche eines Festphasenträgers fixiertes oder bei der Reaktion an den Festphasenträger gebundenes Ende aufweist und das andere Ende mit einer Signalsubstanz gekoppelt ist.

6. Kombinationsprodukt nach Anspruch 3, das ferner einen Teststreifen umfasst,
wobei der Teststreifen ein Probenkissen, eine Reaktionsmembran, versehen mit einer Qualitätskontrollregion und einer Nachweisregion entlang einer Flüssigkeitsflussrichtung und ein Absorptionskissen umfasst;
die Markierung eine erste Markierung und eine zweite Markierung umfasst;
das Probenkissen mit einer Signalsubstanz beschichtet ist, die mit einer ersten Anti-Markierung gegen die erste Markierung markiert ist;
die Qualitätskontrollregion ständig mit einer zweiten Anti-Markierung gegen die zweite Markierung beschichtet ist;
die Nachweisregion ständig mit einem sekundären Antikörper gegen die erste Anti-Markierung beschichtet ist;
die erste Markierung und die erste Anti-Markierung sowie die zweite Markierung und die zweite Anti-Markierung unterschiedliche Markierung-Anti-Markierung-Komplexe ausbilden;
oder der Teststreifen ein Probenkissen, eine Reaktionsmembran, versehen mit einer Qualitätskontrollregion und einer Nachweisregion entlang einer Flüssigkeitsflussrichtung und ein Absorptionskissen umfasst;
die Markierung eine erste Markierung und eine zweite Markierung umfasst;
das Probenkissen mit einer Signalsubstanz beschichtet ist, die mit einer ersten Anti-Markierung gegen die erste Markierung markiert ist;
die Nachweisregion ständig mit einer zweiten Anti-Markierung gegen die zweite Markierung beschichtet ist;
die Qualitätskontrollregion ständig mit einem sekundären Antikörper gegen die erste Anti-Markierung beschichtet ist;
die erste Markierung und die erste Anti-Markierung sowie die zweite Markierung und die zweite Anti-Markierung unterschiedliche Markierung-Anti-Markierung-Komplexe ausbilden.

7. Kombinationsprodukt nach Anspruch 6, wobei die Signalsubstanz aus der Gruppe ausgewählt ist, bestehend aus einem Fluorophor, einer kolorimetrischen Markierung, einem Quantenpunkt, einem kolloidalen Gold, einer Alkingruppe für die Raman-Beugungsbildgebung, einem Cycloalken für die Click-Reaktion, einer Polymermarkierungsstartgruppe, einem Polypeptid/Proteinmolekül, einer LNA/PNA, einer nicht natürlichen Aminosäure und einem Analogon davon, einer nicht natürlichen Nukleinsäure und einem Analogon davon und einer Nanostruktur;
die Nanostruktur ein anorganisches Nanopartikel, ein NV-Zentrum, ein durch Aggregation/Assemblierung induziertes lumineszierendes Molekül, ein Seltenerdionen-Ligandenmolekül und ein Polyoxometallat umfasst.

8. Kombinationsprodukt nach Anspruch 6, wobei die Kombination von Markierung/Anti-Markierung in den Markierung/Anti-Markierung-Komplexen aus der Gruppe ausgewählt ist, bestehend aus Biotin oder einem Derivat davon/Streptavidin, Biotin oder einem Derivat davon/Avidin, Biotin oder Derivat davon/Neutravidin, Hapten/Antikörper, Antigen/Antikörper, Rezeptor/Ligand, Digoxigenin/Digoxigenin-Ligand, Kohlenhydrat/Agglutinin und Polynukleotid/komplementärem Polynukleotid;
wobei das Biotinderivat eines von D-Biotin, aktiviertem Biotin, Biocytin, Ethylendiaminbiotin, Cadaverinbiotin oder Desthiobiotin ist.

9. Kombinationsprodukt nach Anspruch 1, wobei die Sonde ein 3'-Ende aufweist, das einen nicht verlängerbaren Blockierungsteil umfasst.

10. Kombinationsprodukt nach einem der Ansprüche 1 bis 9, wobei die Sonde zwei RNA-Basen aufweist und die komplementäre Region der Sonde eine Länge von weniger als 25 nt aufweist.

11. Kombinationsprodukt nach einem der Ansprüche 1 bis 9, das ferner einen LAMP-Primer, einen NEAR-Primer oder einen RPA-Primer umfasst.

12. Kit, umfassend das Kombinationsprodukt nach einem der Ansprüche 1 bis 11.

13. Verwendung des Kombinationsprodukts nach Anspruch 10 für den Nachweis von SNP, wobei die Sonde zwei RNA-Basen aufweist.

14. Verwendung nach Anspruch 13, wobei bei dem Nachweis für SNP eine Reaktionstemperatur einer Spaltungsreaktion mit der Ribonuklease H II kleiner oder gleich einer Temperatur ist, die in einem Tm-Wert plus 10 °C gezeigt ist, wobei die Reaktionstemperatur der Spaltungsreaktion mit der Ribonuklease H II in einem Bereich von 55 °C-65 °C liegt.

15. Verwendung des Kombinationsprodukts nach einem der Ansprüche 1 bis 9 für den Nachweis einer Ziel-DNA, die aus einer Amplifikation mit einem LAMP-, NEAR- oder RPA-Primer erhalten wurde.

16. Verwendung nach Anspruch 15, wobei jedes Segment der DNA Basen oder Basensubstituenten von weniger als oder gleich 6 aufweist und die Amplifikation bei einer Temperatur von 35 °C bis 45 °C durchgeführt wird.

## Revendications

1. Produit de combinaison pour une détection d'ADN, comprenant une ribonucléase H II et une sonde,
dans lequel la sonde est une sonde simple brin et a une séquence qui est partiellement ou entièrement complémentaire à une molécule d'ADN cible à détecter, et au moins deux bases d'ARN sont intégrées dans la région complémentaire de la sonde et divisent la région complémentaire de la sonde en au moins trois segments, dont chacun a indépendamment 1, 2, 3, 4, 5 ou 6 bases d'ADN et des substitutions de bases qui n'interfèrent pas avec une hybridation d'un brin d'acide nucléique en totalité, et dans lequel les bases d'ARN sont distribuées de façon discrète dans la sonde.

2. Produit de combinaison selon la revendication 1, dans lequel la sonde a une valeur Tm exprimée comme une température qui est supérieure ou égale à une valeur obtenue par soustraction de 10 °C de la température de réaction de la réaction de clivage avec la ribonucléase H II, dans lequel la température de réaction de la réaction de clivage avec la ribonucléase H II se situe dans une plage de 55 °C à 65 °C.

3. Produit de combinaison selon la revendication 1, dans lequel au moins deux segments sont marqués avec différents éléments d'identification détectables, qui sont une séquence d'acide nucléique spécifique ou un marqueur marqué sur des bases et/ou des squelettes d'acide nucléique ; et le marqueur produit un signal détectable lorsque la sonde est clivée au niveau de la base d'ARN avec la ribonucléase H II.

4. Produit de combinaison selon la revendication 3, dans lequel le marqueur comprend un fluorophore et un désactiveur, et au moins un fluorophore et au moins un désactiveur sont situés dans des segments différents.

5. Produit de combinaison selon la revendication 3, dans lequel la sonde a une extrémité fixée à une surface d'un support de phase solide, ou liée au support de phase solide lors d'une réaction, et l'autre extrémité couplée à une substance signal.

6. Produit de combinaison selon la revendication 3, comprenant en outre une bandelette d'essai,
dans lequel la bandelette d'essai comprend un tampon d'échantillon, une membrane de réaction pourvue d'une région d'inspection de qualité et d'une région de détection le long d'une direction d'écoulement de liquide, et un tampon d'absorption ;
le marqueur comprend un premier marqueur et un deuxième marqueur ;
le tampon d'échantillon est recouvert d'une substance signal, qui est marquée avec un premier anti-marqueur contre le premier marqueur ;
la région d'inspection de qualité est constamment recouverte d'un deuxième anti-marqueur contre le deuxième marqueur ;
la région de détection est constamment recouverte d'un anticorps secondaire contre le premier anti-marqueur ;
le premier marqueur et le premier anti-marqueur, ainsi que le deuxième marqueur et le deuxième anti-marqueur, forment différents complexes marqueur-anti-marqueur ;
ou, la bandelette d'essai comprend un tampon d'échantillon, une membrane de réaction pourvue d'une région d'inspection de qualité et d'une région de détection le long d'une direction d'écoulement de liquide, et un tampon d'absorption ;
le marqueur comprend un premier marqueur et un deuxième marqueur ;
le tampon d'échantillon est recouvert d'une substance signal, qui est marqué avec un premier anti-marqueur contre le premier marqueur ;
la région de détection est constamment recouverte d'un deuxième anti-marqueur contre le deuxième marqueur ;
la région d'inspection de qualité est constamment recouverte d'un anticorps secondaire contre le premier anti-marqueur ;
le premier marqueur et le premier anti-marqueur, ainsi que le deuxième marqueur et le deuxième anti-marqueur, forment différents complexes marqueur-anti-marqueur.

7. Produit de combinaison selon la revendication 6, dans lequel la substance signal est sélectionnée dans le groupe constitué d'un fluorophore, d'un marqueur colorimétrique, d'un point quantique, d'un or colloïdal, d'un groupe alcyne pour l'imagerie par diffraction Raman, d'un cycloalcène pour la réaction click, d'un groupe d'initiation de marquage polymère, d'une molécule polypeptidique/protéique, d'un LNA/PNA, d'un acide aminé non naturel et d'analogues de celui-ci, d'un acide nucléique non naturel et d'analogues de celui-ci, et d'une nanostructure ;
la nanostructure comprend une nanoparticule inorganique, un centre NV, une molécule luminescente induite par agrégation/assemblage, une molécule ligand d'ion de terres rares, et un polyoxométalate.

8. Produit de combinaison selon la revendication 6, dans lequel la combinaison du marqueur/anti-marqueur dans les complexes marqueur-anti-marqueur est sélectionnée dans le groupe constitué de biotine ou d'un dérivé de celle-ci/streptavidine, de biotine ou d'un dérivé de celle-ci/avidine, de biotine ou d'un dérivé de celle-ci/neutravidine, d'haptène/anticorps, d'antigène/anticorps, de récepteur/ligand, de digoxigénine/ligand de digoxigénine, de glucide/agglutinine, et de polynucléotide/polynucléotide complémentaire ;
dans lequel le dérivé de biotine est l'une quelconque d'une D-biotine, d'une biotine activée, d'une biocytine, d'une biotine éthylènediamine, d'une biotine cadavérine ou d'une desthiobiotine.

9. Produit de combinaison selon la revendication 1, dans lequel la sonde a une extrémité 3' comprenant une fraction bloquante non extensible.

10. Produit de combinaison selon l'une quelconque des revendications 1 à 9, dans lequel la sonde a deux bases d'ARN, et la région complémentaire de la sonde a une longueur inférieure à 25 nt.

11. Produit de combinaison selon l'une quelconque des revendications 1 à 9, comprenant en outre une amorce LAMP, une amorce NEAR ou une amorce RPA.

12. Kit comprenant le produit de combinaison de l'une quelconque des revendications 1 à 11.

13. Utilisation du produit de combinaison selon la revendication 10 dans la détection de SNP, dans laquelle la sonde comporte deux bases d'ARN.

14. Utilisation selon la revendication 13, dans laquelle, dans la détection de SNP, une température de réaction d'une réaction de clivage avec la ribonucléase H II est inférieure ou égale à une température indiquée dans une valeur Tm plus 10 °C, dans laquelle la température de réaction de la réaction de clivage avec la ribonucléase HII se situe dans une plage de 55 °C à 65 °C.

15. Utilisation du produit de combinaison selon l'une quelconque des revendications 1 à 9 dans la détection d'un ADN cible obtenu à partir d'une amplification avec une amorce LAMP, NEAR ou RPA.

16. Utilisation selon la revendication 15, dans laquelle chaque segment a des bases ou des substituts de base d'ADN inférieurs ou égaux à 6, et l'amplification est réalisée à une température de 35 °C à 45 °C.
